# EUROPEAN PATENT SPECIFICATION

(11) **EP 4 072 643 B1**
(45) Date of publication and mention of the grant of the patent: **07.05.2025**
(21) Application number: 20829477.7
(22) Date of filing: 07.12.2020
(51) Int. Cl.: A61M 25/00, A61M 25/01, A61M 1/00, A61M 1/36, A61M 1/28

(54) **APPARATUS AND METHOD FOR USE OF FLUID FLOW DEVICE**
GERÄT UND VERFAHREN ZUR VERWENDUNG EINER FLÜSSIGKEITSSTRÖMUNGSVORRICHTUNG
APPAREIL ET PROCÉDÉ D'UTILISATION DE DISPOSITIF D'ÉCOULEMENT DE FLUIDE

(30) Priority: 09.12.2019 US 201962945498 P
(43) Date of publication of application: 19.10.2022
(73) Proprietor: The Cleveland Clinic Foundation, Cleveland, OH 44195 (US)
(72) Inventor: MCCURRY, Kenneth, Cleveland, Ohio 44195 (US); BASHOUR, Allen, Cleveland, Ohio 44195 (US); HOVEST, Torey, Cleveland, Ohio 44195 (US); KNOWLTON, Christopher, Cleveland, Ohio 44195 (US); SHOREY, Frederick A., Cleveland, Ohio 44195 (US)
(74) Representative: HGF
(86) International application number: PCT/US2020/063630
(87) International publication number: WO 2021/118933

(56) References cited:
- WO-A1-2018/225331
- WO-A1-2019/066728
- US-A1- 2003 149 395
- US-A1- 2016 008 531
- US-A1- 2019 366 035

## Description

### Related Application

This application claims priority from U.S. Provisional Application No. 62/945,498, filed 9 December 2019.

### Technical Field

This disclosure relates to an apparatus for use of a fluid flow control device and, more particularly, to a cannula apparatus for selective fluid flow in a removal direction and a return direction, opposite the removal direction.

### Background

Extracorporeal membrane oxygenation ("ECMO"), also known as extracorporeal life support, is an extracorporeal technique of providing prolonged cardiac and/or respiratory support to persons whose heart and/or lungs are unable to provide an adequate amount of gas exchange and/or perfusion to sustain life. For example, ECMO could be provided to a patient during or after a major surgical procedure, to help reduce the burden on the patient's own body to oxygenate the blood due either to heart or lung dysfunction, or both.

In ECMO, deoxygenated blood is removed from the vasculature of a patient's body via one lumen of a device, is perfused with oxygen and/or otherwise treated in a therapeutic manner, and then is returned to the patient's vasculature (either through a different lumen of the same device or a different device). US 2003/149395 A1 discloses a cannula apparatus suitable for selective fluid flow in a removal direction and a return direction, comprising the features of the the preamble of claim 1.

### Summary

In an aspect, a cannula apparatus for selective fluid flow in a removal direction and a return direction, opposite the removal direction is described. An outer sheath has proximal and distal outer sheath ends spaced apart by a longitudinal outer sheath body defining an outer sheath lumen. At least one fluid removal aperture extends entirely through the outer sheath body adjacent the distal outer sheath end to place the outer sheath lumen in fluid communication with an ambient space. The outer sheath includes a side access aperture extending entirely through the outer sheath body. An introducer has proximal and distal introducer ends spaced apart by a longitudinal introducer body. The introducer body at least partially defines a guidewire channel longitudinally therealong. The introducer is configured for selective insertion at least partially into the outer sheath lumen with the guidewire channel in fluid communication with the side access aperture. An inner tube has proximal and distal inner tube ends spaced apart by a longitudinal inner tube body defining an inner tube lumen. At least one fluid return aperture is located at least one of at and adjacent the distal inner tube end to place the inner tube lumen in fluid communication with an ambient space. The inner tube is configured for selective insertion into the outer sheath lumen, when the introducer is absent from the outer sheath lumen, with the inner tube body extending completely through the side access aperture to place the distal inner tube end in an ambient space outside the outer sheath.

### Brief Description of the Drawings

For a better understanding, reference may be made to the accompanying drawings, in which:
Fig. 1 is a schematic side view of a first component of a first aspect of the invention;
Fig. 2 is a schematic side view of a second component of the aspect of Fig. 1;
Fig. 3 is a schematic side view of the first and second components of the aspect of Fig. 1 in an assembled state;
Fig. 4 is a cross-sectional view of a second aspect of the invention;
Fig. 5 is a schematic detail view of the aspect of Fig. 4;
Fig. 6 is a schematic side view of the aspect of Fig. 4;
Fig. 7 is a cross-sectional view of a third aspect of the invention;
Fig. 8 is a schematic detail view of the aspect of Fig. 7;
Fig. 9 is a schematic side view of the aspect of Fig. 7;
Fig. 10 is a side view of a first component of a fourth aspect of the invention;
Fig. 11 is a detail of area "11" of Fig. 10;
Fig. 12 is a top view of a second component of the aspect of Fig. 10;
Fig. 13 is a side view of the second component of Fig. 12;
Fig. 14 is a detail of area "14" of Fig. 12;
Fig. 15 is a cross-sectional view of a third component of the aspect of Fig. 10;
Fig. 16 is a top view of the third component of Fig. 15;
Fig. 17 is a cross-section taken along line "17-17" of Fig. 15;
Fig. 18 is a detail of area "18" of Fig. 15;
Fig. 19 is a partial top perspective view of the second and third components of the aspect of Fig. 10 in an assembled state; and
Fig. 20 is a partial perspective view with a cut line along "20-20" of Fig. 19.

### Description of Aspects of the Disclosure

Unless defined otherwise, all technical and scientific terms used herein have the same meaning as is commonly understood by one of skill in the art to which the present disclosure pertains.

As used herein, the term "subject" can be used interchangeably with the term "patient" and refer to any warm-blooded organism including, but not limited to, human beings, pigs, rats, mice, dogs, goats, sheep, horses, monkeys, apes, rabbits, cattle, farm animals, livestock, etc.

As used herein, the singular forms "a," "an" and "the" can include the plural forms as well, unless the context clearly indicates otherwise. It will be further understood that the terms "comprises" and/or "comprising," as used herein, can specify the presence of stated features, steps, operations, elements, and/or components, but do not preclude the presence or addition of one or more other features, steps, operations, elements, components, and/or groups thereof.

As used herein, the term "and/or" can include any and all combinations of one or more of the associated listed items.

It will be understood that when an element is referred to as being "on," "attached" to, "connected" to, "coupled" with, "contacting", "adjacent", etc., another element, it can be directly on, attached to, connected to, coupled with, contacting, or adjacent the other element or intervening elements may also be present. In contrast, when an element is referred to as being, for example, "directly on," "directly attached" to, "directly connected" to, "directly coupled" with, "directly contacting", or "directly adjacent" another element, there are no intervening elements present. It will also be appreciated by those of skill in the art that references to a structure or feature that is disposed "directly adjacent" another feature may have portions that overlap or underlie the adjacent feature, whereas a structure or feature that is disposed "adjacent" another feature might not have portions that overlap or underlie the adjacent feature.

Spatially relative terms, such as "under," "below," "lower," "over," "upper", "proximal", "distal", and the like, may be used herein for ease of description to describe one element or feature's relationship to another element(s) or feature(s) as illustrated in the figures. It will be understood that the spatially relative terms can encompass different orientations of a device in use or operation, in addition to the orientation depicted in the figures. For example, if a device in the figures is inverted, elements described as "under" or "beneath" other elements or features would then be oriented "over" the other elements or features.

As used herein, the phrase "at least one of X and Y" can be interpreted to include X, Y, or a combination of X and Y. For example, if an element is described as having at least one of X and Y, the element may, at a particular time, include X, Y, or a combination of X and Y, the selection of which could vary from time to time. In contrast, the phrase "at least one of X" can be interpreted to include one or more Xs.

It will be understood that, although the terms "first," "second," etc. may be used herein to describe various elements, these elements should not be limited by these terms. These terms are only used to distinguish one element from another. Thus, a "first" element discussed below could also be termed a "second" element without departing from the teachings of the present disclosure. The sequence of operations (or steps) is not limited to the order presented in the claims or figures unless specifically indicated otherwise.

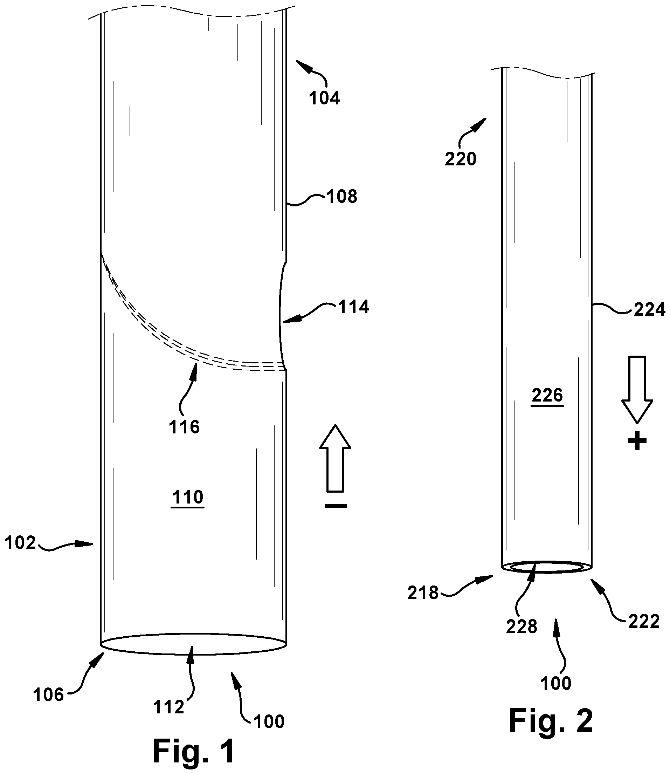

Figs. 1-3 depict a cannula apparatus 100 for selective fluid flow in a removal direction (arrow "-" in the Figures) and in a return direction (arrow "+" in the Figures), opposite the removal direction, within a patient's body. While a vasculature of a patient is used herein as an example use environment, one of ordinary skill in the art will be able to configure any cannula apparatus according to the present invention for a desired use environment (such as, but not limited to, flushing cerebrospinal fluid, ECMO (including pulmonary artery infusion), ECLS, right heart assist or RVAD, wound drainage and flushing, renal dialysis, peritoneal space drainage and flushing, delivery or removal of fluid based therapeutic treatments, and/or other medical and/or non-medical uses).

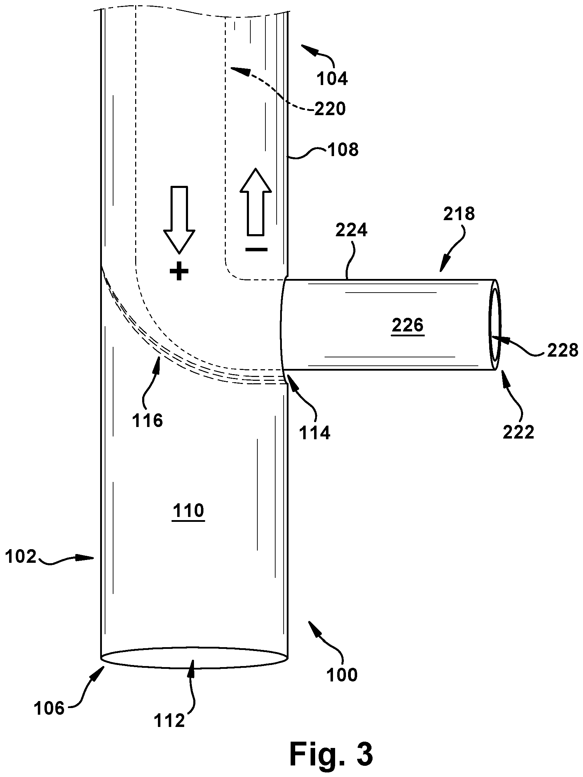

The cannula apparatus 100 includes an outer sheath 102 having proximal and distal outer sheath ends 104 and 106 spaced apart by a longitudinal outer sheath body 108 defining an outer sheath lumen 110. At least one fluid removal aperture 112 extends entirely through the outer sheath body 108 at and/or adjacent the distal outer sheath end 106 to place the outer sheath lumen 110 in fluid communication with an ambient space. As shown in Figs. 1 and 3, the fluid removal aperture 112 is at a distalmost tip of the outer sheath 102 and is simply an open end of the outer sheath body 108 tube. The ambient space within the patient's body may be, for example, an area of the patient's vasculature and/or an internal organ through which blood is flowing. The outer sheath 102 includes a side access aperture 114 and extends entirely through the outer sheath body 108.

A deflector 116 is located within the outer sheath lumen 110 and has any desired configuration operative to steer or bias an inserted structure toward the side access aperture 114. For example, the deflector 116 could be a rail or track which blocks at least a portion of the outer sheath lumen 110 cross-section to prevent a structure of a particular cross-sectional size and/or shape from traveling through the outer sheath lumen 110 past the deflector 116. It is contemplated that, for most use environments of the cannula apparatus 100, the deflector 116 will allow a desired amount of fluid pressure and fluid flow within the outer sheath lumen 110 in the removal direction, optionally in such a manner to minimize turbulent flow of fluid. It is also contemplated that one or more fluid removal apertures (not shown) could be located proximal to the deflector 116.

Fig. 2 depicts an inner tube 218 having proximal and distal inner tube ends 220 and 222 spaced apart by a longitudinal inner tube body 224 defining an inner tube lumen 226. At least one fluid return aperture 228 is located at least one of at and adjacent the distal inner tube end 222 to place the inner tube lumen 226 in fluid communication with an ambient space. The inner tube 218 is configured for selective insertion into the outer sheath lumen 110 with the inner tube body 224 extending completely through the side access aperture 114 of the outer sheath 102 to place the distal inner tube end 222 in an ambient space outside the outer sheath 102. What is meant by "the inner tube body 224 extending completely through the side access aperture 114" is that some portion of the inner tube body 224 passes through the side access aperture 114 during use of the cannula apparatus 100, not that the entirety of the inner tube body 224 is, at some point, passed through the side access aperture 114. The deflector 116 acts to guide or urge the inner tube 218, during its insertion into the outer sheath lumen 110, toward the side access aperture 114. There may optionally be a membrane or other sealing structure (not shown) present within the side access aperture 114 to prevent fluid flow in the removal direction therethrough.

In use, the cannula apparatus 100 of Figs. 1-3 may be utilized to remove fluid from, and concurrently or consecutively return fluid to, a vasculature of a patient. The outer sheath 102 is inserted to a target removal site in the patient's body. The inner tube 218 could be at least partially held within the outer sheath 102 for concurrent insertion, or the inner tube 218 could be absent from the outer sheath 102 until the outer sheath 102 is placed as desired. Once the outer sheath 102 is at the target removal site, the inner tube 218 could be inserted (or further inserted) distally into the outer sheath lumen 110 until the deflector 116 directs the distal inner tube and 222 out the side access aperture 114 of the outer sheath 102, into the configuration shown in Fig. 3 with the fluid return aperture 228 at a target returned site within the patient's vasculature. At that time, the outer sheath 102 can be used to remove fluid from the target removal site, and the inner tube 218 could be used, sequentially and/or simultaneously, to return fluid to the target returned site as desired.

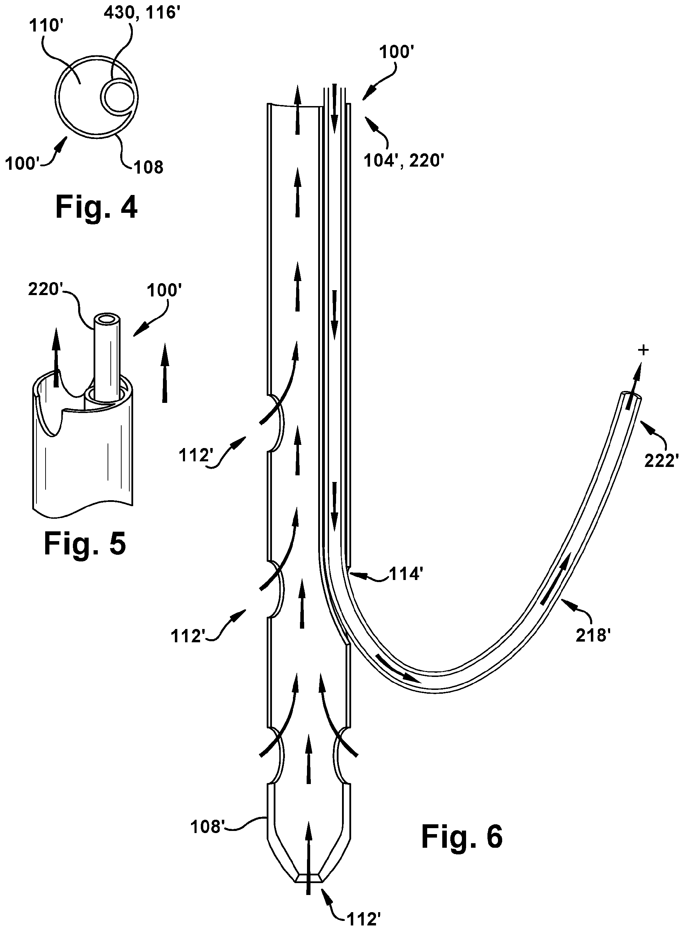

Figs. 4-6 illustrate a second embodiment of a cannula apparatus 100'. The cannula apparatus 100' of Figs. 4-6 is similar to the cannula apparatus 100 of Figs. 1-3 and therefore, structures of Figs. 4-6 that are the same as or similar to those described with reference to Figs. 1-3 have the same reference numbers with the addition of a "prime" mark. Description of common elements and operation similar to those in the previously described first embodiment will not be repeated with respect to the second embodiment, but should instead be considered to be incorporated below by reference as appropriate.

Fig. 4 is a cross-sectional view of the cannula apparatus 100'. Similarly to the first embodiment, the cannula apparatus 100' of the second embodiment includes a deflector 116'. However, in the second embodiment, the deflector 116' takes the form of a channel tube 430 which extends a significant length of the outer sheath body 108', within the outer sheath lumen 110', between the proximal outer sheath end 104' and the side access aperture 114'. The channel tube 430 could be, for example, a smaller-diameter tube attached to an inner wall of the outer sheath body 108'. The inner tube 218' then has its own, "dedicated lane" within the outer sheath lumen 110'. The inner tube 218'can be inserted into the channel tube 430 and out the side access aperture 114' as desired by a user, similarly to the operation of the first embodiment of the cannula apparatus 100'. Stated differently, the second embodiment includes a reduced-diameter outer sheath lumen 110' in cross-sectional areas of the outer sheath body 108' through which the inner tube 218' extends within the channel tube 430.

It should also be noted, for the sake of illustration, that the outer sheath 102' of the second embodiment includes a plurality of fluid removal apertures 112' at differing longitudinal spacing along the outer sheath body 108'. As with any embodiment of the present invention, there may be any number, location(s), and size(s) of fluid removal apertures 112' as desired for a particular use environment in order to remove or "drain" fluid appropriately. For example, in an ECMO use environment, the fluid removal apertures 112' could be configured to drain about one-third of the total removal volume from the head/SVC and about two-thirds from the IVC.

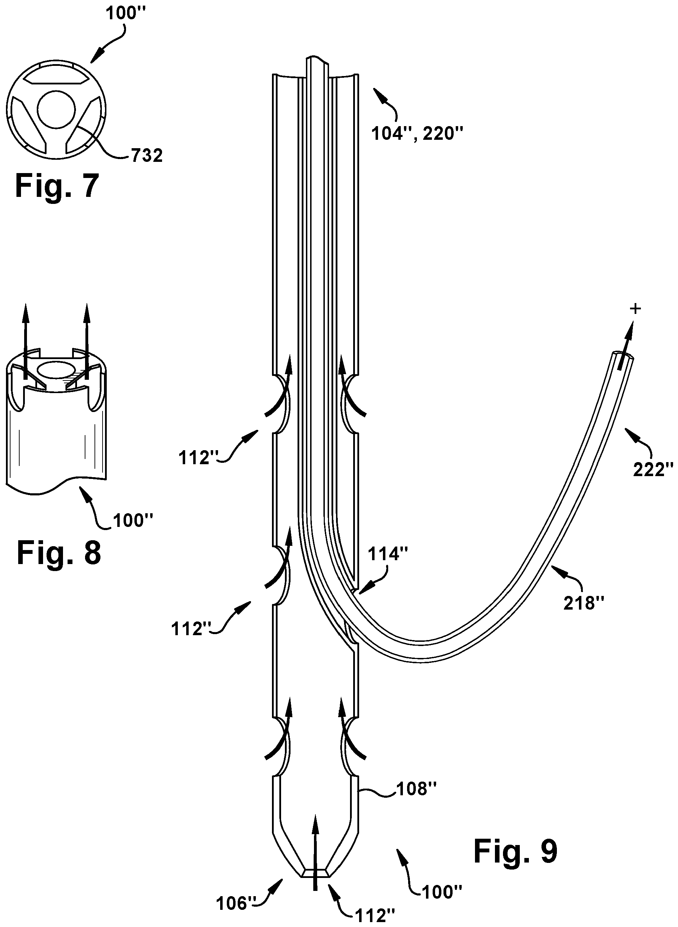

Figs. 7-9 illustrate a third embodiment of a cannula apparatus 100". The cannula apparatus 100" of Figs. 7-9 is similar to the cannula apparatuses 100, 100' of Figs. 1-6 and therefore, structures of Figs. 7-9 that are the same as or similar to those described with reference to Figs. 1- 6 have the same reference numbers with the addition of a double "prime" mark. Description of common elements and operation similar to those in the previously described first and second embodiments will not be repeated with respect to the third embodiment, but should instead be considered to be incorporated below by reference as appropriate.

In the third embodiment of the cannula apparatus 100", as shown in Fig. 7, the deflector 116 takes the form of a spacer frame 732 which holds the inner tube 218" away from the outer sheath body 108" walls within the outer sheath lumen 110". It is contemplated that one or more spacer frames 732 could be provided along the length of the outer sheath body 108" , in order to "suspend" the inner tube 218" within the outer sheath lumen". That is, fluid is removed concentrically around the inner tube 218" through the outer sheath 102"during use of the cannula apparatus 100" of the third embodiment. The inner tube 218" could be directed toward the side access aperture 114" in any desired manner, such as, but not limited to, a spacer frame 732 configured to urge the inner tube 218" and/or a steerable feature of the inner tube 218".

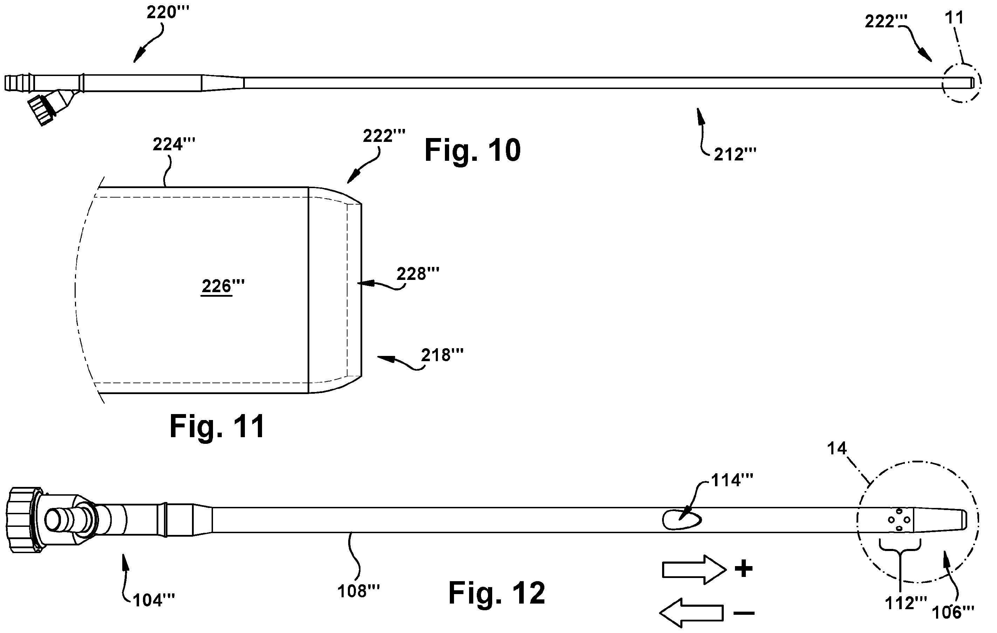

Figs. 10-20 illustrate a fourth embodiment of a cannula apparatus 100"'. The cannula apparatus 100‴ of Figs. 10-20 is similar to the cannula apparatuses 100, 100', 100" of Figs. 1- 9 and therefore, structures of Figs. 10-20 that are the same as or similar to those described with reference to Figs. 1-9 have the same reference numbers with the addition of a triple "prime" mark. Description of common elements and operation similar to those in the previously described first through third embodiments will not be repeated with respect to the fourth embodiment, but should instead be considered to be incorporated below by reference as appropriate.

The cannula apparatus 100‴ shown in Figs. 10-20 helps provide selective fluid flow in both a removal direction and a return direction, opposite the removal direction, within a vasculature of a patient. As shown in Figs. 10-20, the outer sheath 102"', inner tube 218"', and other structures of the cannula apparatus 100‴ of the fourth embodiment may include connectors, couplers, control features, and/or other structures used to attach the components of the cannula apparatus 100‴ to one another and/or to other, related devices. One of ordinary skill in the art can readily configure a cannula apparatus 100"', and components thereof, for a particular use environment.

The cannula apparatus 100‴ of the fourth embodiment includes an outer sheath 102‴ having proximal and distal outer sheath ends 104‴ and 106‴ spaced apart by a longitudinal outer sheath body 108‴ defining an outer sheath lumen 110‴. At least one fluid removal aperture 112‴ extends entirely through the outer sheath body adjacent the distal outer sheath end 106‴ to place the outer sheath lumen 110‴ in fluid communication with an ambient space. As shown in Figs. 12-13, the outer sheath 102 includes a side access aperture 114‴ extending entirely through the outer sheath body 108"'. The side access aperture 114‴ may be located proximal to all fluid removal apertures 112"', or there may be one or more fluid removal apertures 112‴ located proximal to the side access aperture 114"'. The distal outer sheath end 106‴ and/or the distal inner tube end 222‴ may include a solid, nonapertured distalmost tip. Alternatively, the distal most tip of the outer sheath 110‴ and/or the inner tube 218‴ could be open, such as by a simple operation of the respective lumen ending at a cut-off the rim of the tube structure.

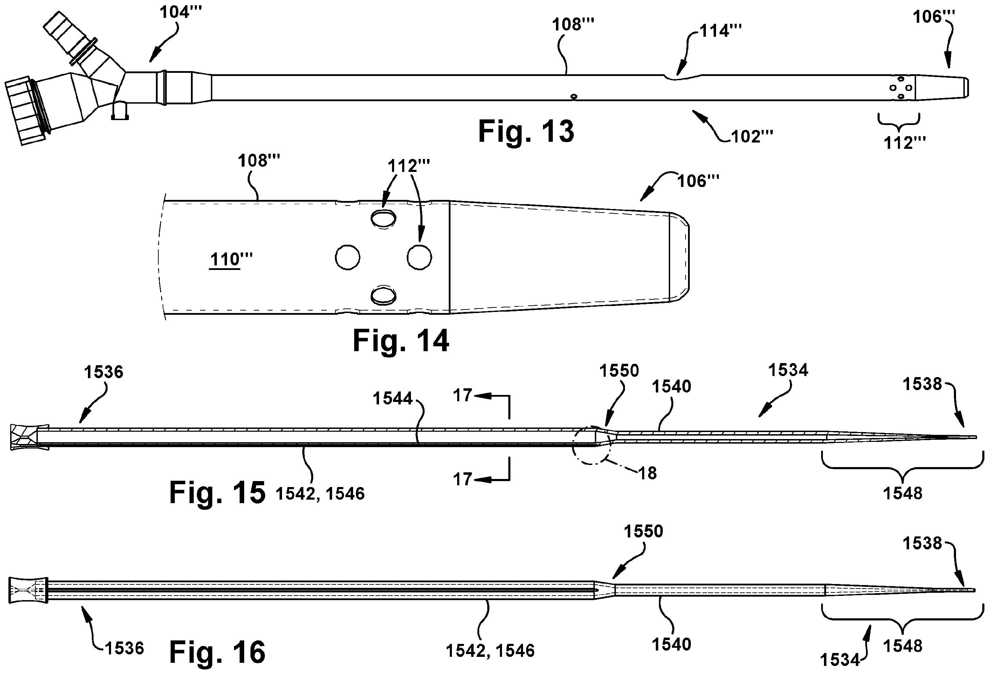

As shown in Figs. 15-16, the cannula apparatus 100‴ of the fourth embodiment may include an introducer 1534 having proximal and distal introducer ends 1536 and 1538 spaced apart by a longitudinal introducer body 1540. The introducer body 1540 at least partially defines a guidewire channel 1542 longitudinally therealong, which may assist with placement of at least a portion of the cannula apparatus 100‴. For example, at least one of the introducer 1534 and the outer sheath 110‴ may be present in a desired location, and then a balloon tipped catheter (serving as a guidewire) could be inserted through the "guidewire channel" in the introducer body 1540. Once the catheter distally exits the channel of the introducer body 1540, the balloon is inflated and the catheter is floated past the TV into the RV, through the RVOT and into the PA. The balloon tipped catheter could then be exchanged for a guidewire (without dislodging the guidewire as the catheter is withdrawn and/or as the introducer 1534 is removed) over which the inner tube 218‴ may be inserted and the guidewire removed. Alternately, it is contemplated that the guidewire may be placed first via the guidewire channel 1542, without the use of a balloon tipped catheter, in certain embodiments, or the guidewire may be placed first and backloaded into the guidewire channel 1542, in other embodiments. One of ordinary skill in the art will be able to readily substitute or supplement the guidewire, in the instant description, for a balloon tipped catheter, for a particular use environment.

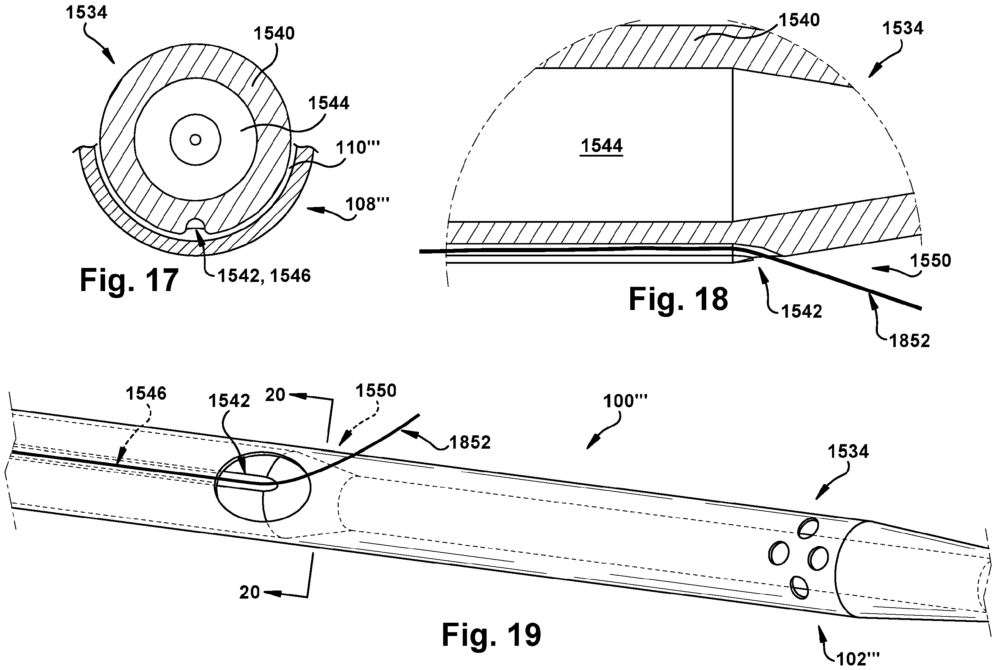

The introducer 1534 is configured for selective insertion at least partially into the outer sheath lumen 110‴ with the guidewire channel 1542 in fluid communication with the side access aperture 114‴. As shown in the Figures, the introducer body 1540 may include a guidewire groove 1546 extending partially into the introducer body 1540 from a laterally outermost surface thereof. In this case, and innermost wall of the outer sheath body 108‴ will at least partially define, in cooperation with the guidewire groove 1546 of the introducer body 1540, the guidewire channel 1542 when the introducer 1534 is at least partially located within the outer sheath lumen 110"'. This is the situation shown schematically in Fig. 17, with only a portion of the circumference of the outer sheath body 108‴ shown, for clarity. As an alternative configuration, though not shown, the guidewire channel 1542 could be a longitudinally extending "tunnel" provided within the introducer body 1540, such as by being a side lumen completely cross-sectionally enclosed by the structure of the introducer body 1540.

As shown in Figs. 15-16, the introducer body 1540 may be contoured along a longitudinal length thereof from a first cross-sectional area at the proximal introducer end 1536 to a reduced second cross-sectional area at the distal introducer end 1538. This taper or reduced-diameter contour could be accomplished with a substantially constant reduction in diameter along the introducer body 1540, or, as shown in the Figures, the introducer body 1540 may have a tapered distal portion 1548 which, when the introducer 1534 is at least partially located within the outer sheath lumen 110"', protrudes distally from the outer sheath 102‴ through an open portion of the distal outer sheath end 106"'. In this case, the tapered distal portion 1548 can act to help open up the vasculature of the patient, much in the manner of a traditional introducer, and ease the increased-diameter passage of the outer sheath 102‴ without subjecting the vasculature to the relatively blunt distal outer sheath and 106‴.

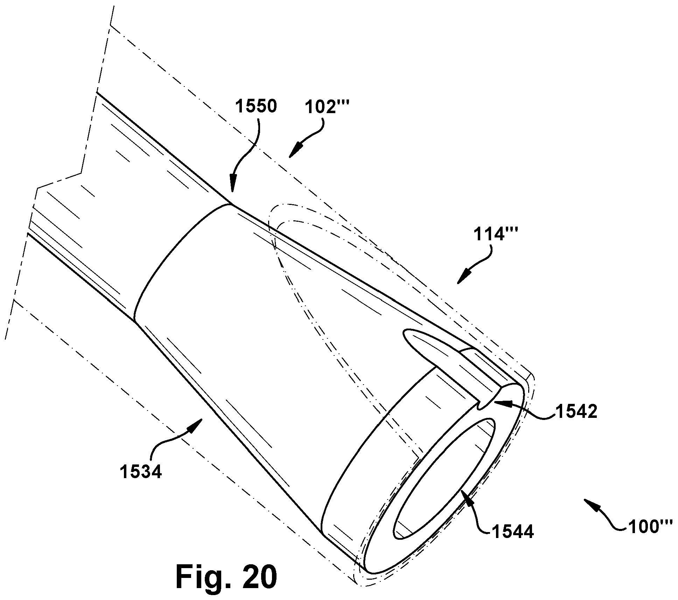

Also as shown in Figs. 15-16, the introducer body 1540, when at least partially located within the outer sheath lumen 110"', may have a substantially constant first cross-sectional area along a length thereof located proximal to the side access aperture 114‴ of the outer sheath 102‴, and the reduction to the second cross-sectional area occurs at least one of laterally adjacent to and distal to the side access aperture 114‴ of the outer sheath 102"'. Stated differently, and as shown in the Figures, an intermediate taper 1550 may occur in the introducer 1534 in approximate longitudinal alignment with the portion of the outer sheath 102‴ which includes the side access aperture 114"', when the introducer 1534 is in place within the outer sheath lumen 110"'. This arrangement is shown in magnified view in Fig. 19 and in the cross-sectional detail view of Fig. 20, and, when present, this intermediate taper 1550 may assist with avoiding interference with an already-placed guidewire during withdrawal of the introducer body 1540. It is contemplated, though, that a substantially constant-cross-section introducer body 1540 could be instead provided, optionally including a longitudinal groove or notch therein to facilitate maintenance of the guidewire in place therein (i.e., avoiding dragging the guidewire backwards) when the introducer 1534 is withdrawn in the proximal direction from the outer sheath lumen 110‴. It is also contemplated that, when the introducer 1534 is at least partially located within the outer sheath lumen 110"', the distal introducer end 1538 could be located a chosen one of laterally adjacent to, and distally to, the side access aperture 114‴ of the outer sheath 102‴

As shown in Figs. 15-16, the introducer 1534 may be at least partially hollow to define an introducer lumen 1544, for any desired reason, such as, but not limited to, flexibility of the introducer 1534 structure.

An inner tube 218‴ has having proximal and distal inner tube ends 220‴ and 222‴ spaced apart by a longitudinal inner tube body 224‴ defining an inner tube lumen 226‴. At least one fluid return aperture 228‴ is located at least one of at and adjacent the distal inner tube end 222‴ to place the inner tube lumen 226‴ in fluid communication with an ambient space. The inner tube 218‴ is configured for selective insertion into the outer sheath lumen 110"', when the introducer 1534 is absent from the outer sheath lumen 110‴, with the inner tube body 224‴ (or portions thereof, as noted above) extending completely through the side access aperture 114‴ to place the distal inner tube end 106‴ in an ambient space outside the outer sheath 102‴.

The inner tube 218‴ might include only one fluid return aperture 228"'. Further, and as shown in Figs. 10-11, that one fluid return aperture 228‴ may be located at a distalmost extent of the inner tube 218"'. However, the inner tube 218‴ may also or instead include at least one fluid return aperture (not shown in the Figures) located adjacent to, but not at, the distal inner tube end 222‴.

As shown schematically in Fig. 18, a guidewire 1852 may be configured for selective introduction through the guidewire channel 1542 while the introducer 1534 is at least partially located within the outer sheath lumen 110"'. A distalmost end of the guidewire 1852 may egress the outer sheath lumen 110‴ via the side access aperture 114‴. That is, and as shown schematically in Fig. 19, the guidewire 1852 may travel along the guidewire channel 1542, which itself may be formed collectively by the guidewire groove 1546 and innermost wall of the outer sheath body 108"', as shown in Fig. 17. Then, when the guidewire 1852 reaches the side access aperture 114"', it may be directed laterally outward through the side access aperture 114"', where it may later be used to guide placement of the inner tube 218"', as will be discussed in detail below.

A method, not forming part of the present invention, of placing a cannula apparatus 100‴ for selective fluid flow in a removal direction and a return direction, opposite the removal direction, within a vasculature of a patient will now be described, using the fourth embodiment, of Figs. 10-20, as an example. The cannula apparatus 100‴ as described above is provided. The introducer 1534 is introduced at least partially into the outer sheath 102‴. With the introducer 1534 maintained at least partially within the outer sheath lumen 110‴, and optionally inserted therein to sufficiently to protrude distally from the distal outer sheath and 106"', whether or not the introducer 1534 is tapered as described, the distal outer sheath and 106‴ and distal introducer and 1538 are advanced to a predetermined target removal location within the vasculature of the patient.

The distal outer sheath end 106‴ and distal introducer and 1538 are maintained at the target removal location, once achieved. When a guidewire 1852 is used, the outer sheath 102‴ and/or the introducer 1534 maintained there in our adjusted to place the guidewire channel 1542 in fluid communication with the side access aperture 114"'. This step could be performed whether the guidewire channel 1542 is of the tunnel type, through the wall of the introducer body 1540, or whether the guidewire channel 1542 is collectively formed by the guidewire groove 1546 and the innermost wall of the outer sheath body 108"'. Once the guidewire channel 1542 is aligned in fluid communication with the side access aperture 114"', as shown in Figs. 19-20, the guidewire 1852 may be advanced distally through the guidewire channel 1542.

The guidewire 1852 is then extended completely through the side access aperture 114‴ (by "completely through" as previously noted, at least some portion of the guidewire 1852 is concurrently located on both and inside and outside of the outer sheath lumen 110‴ and penetrates through the side access aperture 114‴). This places a distal guidewire end adjacent a target return location in the vasculature outside the outer sheath 102"'.

The introducer 1534 can then be withdrawn from the outer sheath 102‴ while the distal guidewire end is maintained adjacent a target return location, and the distal outer sheath and 106‴ is maintained at the target removal location.

When a guidewire 1852 is not being used, once the distal outer sheath end 106‴ and distal introducer end 1538 have achieved their placement at the target removal location, the introducer 1534 can be withdrawn from the outer sheath 102"'. The inner tube 218‴ is then inserted into the outer sheath lumen 110‴ (with the introducer 1534 absent therefrom) and advanced therethrough. The distal inner tube end 222‴ is steered, in any desired manner, completely through the side access aperture 114‴ to a placement at a target return location of the vasculature outside the outer sheath 102‴.

When a guidewire 1852 is being used, however, steering of the inner tube 218‴ toward, and through, the side access aperture 114‴ may be simplified. As above, the guidewire 1852 is left extending through the side access aperture 114‴ when the introducer 1534 is withdrawn. In this case, the inner tube 218‴ is manipulated to place the guidewire 1852 within the inner tube lumen 226"', and the inner tube 218‴ may then be passed along the previously-placed guidewire 1852, as if along a rail, and the distal inner tube and 222‴ will be guided directly to, and out, the side access aperture 114‴ into the ambient space of the vascular outside the outer sheath 102‴. The guidewire 1852 may then be removed as desired from the outer sheath 102‴ with the inner tube body 224‴ maintained through the side access aperture 114"'.

Once emplaced as just described, the outer sheath 102‴ and inner tube 218‴ can be adjusted as desired, and then fluid can be selectively removed from the target removal location through the outer sheath lumen 110"', and, more specifically, through at least one fluid removal aperture 112"'. Likewise, and concurrently or consecutively (or both at different times during operation of the cannula apparatus 100), fluid can be selectively returned to the target return location through the inner sheath lumen 226‴ and, more specifically through at least one fluid return aperture 228‴ thereof.

Particularly when the cannula apparatus 100 is being used for an ECMO procedure, or any other filtering or fluid treatment procedure, the removed fluid could be therapeutically treated (e.g., could be oxygenated and/or filtered) after its removal from the vasculature and before its return to the vasculature. Any desired treatment devices, with appropriate connectors, could be provided to, and/or used with, the cannula apparatus 100 as desired. For example, both the outer sheath 102 and inner tube 218 could have hubs provided with two ports each (one for draining or infusing, and the other to allow over-the-wire/catheter movement, and in the case of the outer sheath 102, to allow the inner tube 218 to be inserted). That is, for the inner tube 218, by having two ports, a user can move the inner tube 218 without disconnecting the blood flow to it, so a two-port hub will help facilitate adjustability over a wire/catheter of the device in-situ while still infusing blood.

While aspects of this disclosure have been particularly shown and described with reference to the example aspects above, it will be understood by those of ordinary skill in the art that various additional aspects may be contemplated. In an effort to maintain clarity in the Figures, certain ones of duplicative components shown have not been specifically numbered, but one of ordinary skill in the art will realize, based upon the components that were numbered, the element numbers which should be associated with the unnumbered components; no differentiation between similar components is intended or implied solely by the presence or absence of an element number in the Figures. Any of the described structures and components could be integrally formed as a single unitary or monolithic piece or made up of separate sub-components, with either of these formations involving any suitable stock or bespoke components and/or any suitable material or combinations of materials; however, the chosen material(s) should be biocompatible for many applications. Any of the described structures and components could be disposable or reusable as desired for a particular use environment. Any component could be provided with a user-perceptible marking to indicate a material, configuration, at least one dimension, or the like pertaining to that component, the user-perceptible marking potentially aiding a user in selecting one component from an array of similar components for a particular use environment. A "predetermined" status may be determined at any time before the structures being manipulated actually reach that status, the "predetermination" being made as late as immediately before the structure achieves the predetermined status. The term "substantially" is used herein to indicate a quality that is largely, but not necessarily wholly, that which is specified--a "substantial" quality admits of the potential for some relatively minor inclusion of a non-quality item. Though certain components described herein are shown as having specific geometric shapes, all structures of this disclosure may have any suitable shapes, sizes, configurations, relative relationships, cross-sectional areas, or any other physical characteristics as desirable for a particular application. Any structures or features described with reference to one aspect or configuration could be provided, singly or in combination with other structures or features, to any other aspect or configuration, as it would be impractical to describe each of the aspects and configurations discussed herein as having all of the options discussed with respect to all of the other aspects and configurations. A device incorporating any of these features should be understood to fall under the scope of this disclosure as determined based upon the claims below.

Other aspects, objects, and advantages can be obtained from a study of the drawings, the disclosure, and the appended claims.

## Claims

1. A cannula apparatus (100) for selective fluid flow in both a removal direction and a return direction, opposite the removal direction, the apparatus comprising:
an outer sheath (102) having proximal and distal outer sheath ends (104, 106) spaced apart by a longitudinal outer sheath body (108) defining an outer sheath lumen (110), at least one fluid removal aperture (112) extending entirely through the outer sheath body adjacent the distal outer sheath end to place the outer sheath lumen in fluid communication with an ambient space, the outer sheath including a side access aperture (114) extending entirely through the outer sheath body wall;
an introducer (1534) having proximal and distal introducer ends (1536, 1538) spaced apart by a longitudinal introducer body (1540), and
an inner tube (218) having proximal and distal inner tube ends (220, 222) spaced apart by a longitudinal inner tube body (224) defining an inner tube lumen (226), at least one fluid return aperture (228) located at least one of at and adjacent the distal inner tube end to place the inner tube lumen in fluid communication with an ambient space, the inner tube being configured for selective insertion into the outer sheath lumen, when the introducer is absent from the outer sheath lumen, with the inner tube body extending completely through the side access aperture to place the distal inner tube end in an ambient space outside the outer sheath,
**characterized in that** the introducer body at least partially defines a guidewire channel (1542) longitudinally therealong, and **in that** the introducer is configured for selective insertion at least partially into the outer sheath lumen with the guidewire channel in fluid communication with the side access aperture.

2. The apparatus of claim 1, wherein the distal outer sheath end has a solid, nonapertured distalmost tip.

3. The apparatus of claim 1 or 2, wherein the introducer body includes a guidewire groove (1546) extending partially into the introducer body from a laterally outermost surface thereof, an innermost wall of the outer sheath body at least partially defining, in cooperation with the guidewire groove of the introducer body, the guidewire channel when the introducer is at least partially located within the outer sheath lumen.

4. The apparatus of any preceding claim, including a guidewire (1852) configured for selective introduction through the guidewire channel while the introducer is at least partially located within the outer sheath lumen, a distalmost end of the guidewire egressing the outer sheath lumen via the side access aperture.

5. The apparatus of any preceding claim, wherein the introducer body is contoured along a longitudinal length thereof from a first cross-sectional area at the proximal introducer end to a reduced second cross-sectional area at the distal introducer end.

6. The apparatus of claim 5, wherein the introducer body, when at least partially located within the outer sheath lumen, has a substantially constant first cross-sectional area along a length thereof located proximal to the side access aperture of the outer sheath and the reduction to the second cross-sectional area occurs at least one of laterally adjacent to and distal to the side access aperture of the outer sheath.

7. The apparatus of claim 5, wherein the introducer body has a tapered distal portion (1548) which, when the introducer is at least partially located within the outer sheath lumen, protrudes distally from the outer sheath through an open portion of the distal outer sheath end.

8. The apparatus of any of claims 1 to 5, wherein, when the introducer is at least partially located within the outer sheath lumen, the distal introducer end is located a chosen one of laterally adjacent to and distally to the side access aperture of the outer sheath.

9. The apparatus of any of claims 1 to 5 or 8, wherein the inner tube includes only one fluid return aperture (228").

10. The apparatus of claim 9, wherein the one fluid return aperture is located at a distalmost extent of the inner tube.

11. The apparatus of any of claims 1 to 5 or 8 or 9, wherein the inner tube includes at least one fluid return aperture (228), located adjacent to the distal inner tube end.

12. The apparatus of any of claims 1 to 5 or 8 or 9 or 11, wherein the guidewire comprises a balloon tipped catheter.

## Patentansprüche

1. Kanülengerät (100) zur selektiven Flüssigkeitsströmung sowohl in einer Entnahmerichtung als auch in einer Rückführrichtung, entgegengesetzt zur Entnahmerichtung, wobei das Gerät umfasst:
eine Außenhülle (102) mit proximalem und distalem Außenhüllenende (104, 106), die durch einen längsverlaufenden Außenhüllenkörper (108) voneinander beabstandet sind, der ein Außenhüllenlumen (110) definiert, wobei sich zumindest eine Flüssigkeitsentnahmeöffnung (112) vollständig durch den Außenhüllenkörper angrenzend an das distale Außenhüllenende erstreckt, um das Außenhüllenlumen in Flüssigkeitsverbindung mit einem Umgebungsraum zu bringen, wobei die Außenhülle eine Seitenzugangsöffnung (114) beinhaltet, die sich vollständig durch die Außenhüllenkörperwand erstreckt;
einen Einführer (1534) mit proximalem und distalem Einführerende (1536, 1538), die durch einen längsverlaufenden Einführerkörper (1540) voneinander beabstandet sind,
und
ein Innenrohr (218) mit proximalem und distalem Innenrohrende (220, 222), die durch einen längsverlaufenden Innenrohrkörper (224) voneinander beabstandet sind, der ein Innenrohrlumen (226) definiert, wobei sich zumindest eine Flüssigkeitsrückführöffnung (228) zumindest eines von an oder benachbart zu dem distalen Innenrohrende befindet, um das Innenrohrlumen in Flüssigkeitsverbindung mit einem Umgebungsraum zu bringen, wobei das Innenrohr zum selektiven Einführen in das Außenhüllenlumen konfiguriert ist, wenn der Einführer in dem Außenhüllenlumen fehlt, wobei sich der Innenrohrkörper komplett durch die Seitenzugangsöffnung erstreckt, um das distale Innenrohrende in einen Umgebungsraum außerhalb der Außenhülle zu bringen, **dadurch gekennzeichnet, dass** der Einführerkörper zumindest teilweise einen Führungsdrahtkanal (1542) in Längsrichtung entlang desselben definiert, und dass der Einführer zur selektiven Einführung zumindest teilweise in das Außenhüllenlumen konfiguriert ist, wobei der Führungsdrahtkanal in Flüssigkeitsverbindung mit der Seitenzugangsöffnung steht.

2. Gerät nach Anspruch 1, wobei das distale Außenhüllenende eine feste, ungeöffnete distalste Spitze aufweist.

3. Gerät nach Anspruch 1 oder 2, wobei der Einführerkörper eine Führungsdrahtnut (1546) beinhaltet, die sich von einer seitlich äußersten Oberfläche davon teilweise in den Einführerkörper erstreckt, wobei eine innerste Wand des Außenhüllenkörpers im Zusammenwirken mit der Führungsdrahtnut des Einführerkörpers zumindest teilweise den Führungsdrahtkanal definiert, wenn sich der Einführer zumindest teilweise innerhalb des Außenhüllenlumens befindet.

4. Gerät nach einem der vorhergehenden Ansprüche, beinhaltend einen Führungsdraht (1852), der zur selektiven Einführung durch den Führungsdrahtkanal konfiguriert ist, während sich der Einführer zumindest teilweise innerhalb des Außenhüllenlumens befindet, wobei ein distalstes Ende des Führungsdrahts aus dem Außenhüllenlumen über die Seitenzugangsöffnung austritt.

5. Gerät nach einem der vorhergehenden Ansprüche, wobei der Einführerkörper entlang einer längsverlaufenden Länge davon von einer ersten Querschnittsfläche am proximalen Einführerende zu einer verringerten zweiten Querschnittsfläche am distalen Einführerende konturiert ist.

6. Gerät nach Anspruch 5, wobei der Einführerkörper, wenn er sich zumindest teilweise innerhalb des Außenhüllenlumens befindet, eine im Wesentlichen konstante erste Querschnittsfläche entlang einer Länge davon aufweist, die sich proximal zu der Seitenzugangsöffnung der Außenhülle befindet, und die Verringerung auf die zweite Querschnittsfläche zumindest eines von seitlich benachbart zu oder distal zu der Seitenzugangsöffnung der Außenhülle erfolgt.

7. Gerät nach Anspruch 5, wobei der Einführerkörper einen sich verjüngenden distalen Abschnitt (1548) aufweist, der, wenn sich der Einführer zumindest teilweise innerhalb des Außenhüllenlumens befindet, distal von der Außenhülle durch einen offenen Abschnitt des distalen Außenhüllenendes vorsteht.

8. Gerät nach einem der Ansprüche 1 bis 5, wobei, wenn sich der Einführer zumindest teilweise innerhalb des Außenhüllenlumens befindet, sich das distale Einführerende entweder seitlich benachbart zu oder distal zu der Seitenzugangsöffnung der Außenhülle befindet.

9. Gerät nach einem der Ansprüche 1 bis 5 oder 8, wobei das Innenrohr nur eine Flüssigkeitsrückführöffnung (228") beinhaltet.

10. Gerät nach Anspruch 9, wobei sich die eine Flüssigkeitsrückführöffnung an einer distalsten Ausdehnung des Innenrohrs befindet.

11. Gerät nach einem der Ansprüche 1 bis 5 oder 8 oder 9, wobei das Innenrohr zumindest eine Flüssigkeitsrückführöffnung (228) beinhaltet, die sich benachbart zu dem distalen Innenrohrende befindet.

12. Gerät nach einem der Ansprüche 1 bis 5 oder 8 oder 9 oder 11, wobei der Führungsdraht einen Katheter mit Ballonspitze umfasst.

## Revendications

1. Appareil à canule (100) destiné à un écoulement sélectif de fluide à la fois dans une direction d'évacuation et dans une direction de retour, opposée à la direction d'évacuation, l'appareil comprenant :
une gaine externe (102) comportant des extrémités proximale et distale (104, 106) de gaine externe espacées par un corps longitudinal (108) de gaine externe définissant une lumière (110) de gaine externe, au moins une ouverture d'évacuation de fluide (112) s'étendant entièrement à travers le corps de gaine externe adjacente à l'extrémité de gaine externe distale pour placer la lumière de gaine externe en communication fluide avec un espace ambiant, la gaine externe comprenant une ouverture d'accès latérale (114) s'étendant entièrement à travers la paroi de corps de gaine externe ; un introducteur (1534) comportant des extrémités proximale et distale (1536, 1538) d'introducteur espacées par un corps longitudinal (1540) d'introducteur,
et
un tube interne (218) comportant des extrémités proximale et distale (220, 222) de tube interne espacées par un corps longitudinal (224) de tube interne définissant une lumière (226) de tube interne, au moins une ouverture de retour de fluide (228) étant situé à au moins un emplacement parmi un emplacement au niveau de l'extrémité de tube interne distale et un emplacement adjacent à celle-ci pour placer la lumière de tube interne en communication fluide avec un espace ambiant, le tube interne étant conçu pour une insertion sélective dans la lumière de gaine externe, lorsque l'introducteur est absent de la lumière de gaine externe, le corps de tube interne s'étendant complètement à travers l'ouverture d'accès latérale pour placer l'extrémité de tube interne distale dans un espace ambiant à l'extérieur de la gaine externe, **caractérisé en ce que** le corps d'introducteur définit au moins partiellement un canal de fil-guide (1542) longitudinalement le long de celui-ci, et **en ce que** l'introducteur est conçu pour une insertion sélective au moins partiellement dans la lumière de gaine externe avec le canal de fil-guide en communication fluide avec l'ouverture d'accès latérale.

2. Appareil de la revendication 1, ladite extrémité de gaine externe distale présentant une pointe la plus distale pleine, non ouverte.

3. Appareil de la revendication 1 ou 2, ledit corps d'introducteur comprenant une rainure de fil-guide (1546) s'étendant partiellement dans le corps d'introducteur à partir d'une surface latéralement la plus à l'extérieur de celui-ci, une paroi la plus à l'intérieur du corps de gaine externe définissant au moins partiellement, en coopération avec la rainure de fil-guide du corps d'introducteur, le canal de fil-guide lorsque l'introducteur est au moins partiellement situé à l'intérieur de la lumière de gaine externe.

4. Appareil d'une quelconque revendication précédente, comprenant un fil-guide (1852) conçu pour une introduction sélective à travers le canal de fil-guide pendant que l'introducteur est au moins partiellement situé à l'intérieur de la lumière de gaine externe, une extrémité la plus distale du fil-guide sortant de la lumière de gaine externe par l'intermédiaire de l'ouverture d'accès latérale.

5. Appareil d'une quelconque revendication précédente, ledit corps d'introducteur étant profilé le long d'une longueur longitudinale de celui-ci à partir d'une première section transversale au niveau de l'extrémité proximale d'introducteur jusqu'à une seconde section transversale réduite au niveau de l'extrémité distale d'introducteur.

6. Appareil de la revendication 5, ledit corps d'introducteur, lorsqu'il est au moins partiellement situé à l'intérieur de la lumière de gaine externe, comportant une première section transversale sensiblement constante le long d'une longueur de celui-ci située à proximité de l'ouverture d'accès latéral de la gaine externe et la réduction à la seconde section transversale se produisant au moins un emplacement parmi un emplacement latéralement adjacent à l'ouverture d'accès latéral de la gaine externe et un emplacement distale à celle-ci.

7. Appareil de la revendication 5, ledit corps d'introducteur comportant une partie distale conique (1548) qui, lorsque l'introducteur est au moins partiellement situé à l'intérieur de la lumière de gaine externe, fait saillie de manière distale à partir de la gaine externe à travers une partie ouverte de l'extrémité de gaine externe distale.

8. Appareil de l'une quelconque des revendications 1 à 5, lorsque l'introducteur est au moins partiellement situé à l'intérieur de la lumière de gaine externe, ladite extrémité d'introducteur distale étant située d'une façon choisie parmi latéralement adjacent à l'ouverture d'accès latéral de la gaine externe et distale par rapport à celle-ci.

9. Appareil de l'une quelconque des revendications 1 à 5 ou 8, ledit tube interne comprenant uniquement une ouverture de retour de fluide (228").

10. Appareil de la revendication 9, ladite ouverture de retour de fluide étant située au niveau d'une étendue la plus distale du tube interne.

11. Appareil de l'une quelconque des revendications 1 à 5 ou 8 ou 9, ledit tube interne comprenant au moins une ouverture de retour de fluide (228), située adjacente à l'extrémité distale de tube interne.

12. Appareil de l'une quelconque des revendications 1 à 5 ou 8 ou 9 ou 11, ledit fil-guide comprenant un cathéter à pointe à ballonnet.
